# EUROPEAN PATENT SPECIFICATION

(11) **EP 2 051 721 B1**
(45) Date of publication and mention of the grant of the patent: **10.10.2018**
(21) Application number: 07763808.8
(22) Date of filing: 11.07.2007
(51) Int. Cl.: A61P 9/12, A61K 36/889

(54) **PREPARATION AND PHARMACEUTICAL USE OF EUTERPE OLERACEA (ACAI) EXTRACT COMPOSITIONS**
ZUBEREITUNG UND PHARMAZEUTISCHE VERWENDUNG VON ZUSAMMENSETZUNGEN AUS DEM EXTRAKT VON EUTERPE OLERACEA (ACAI)
PRÉPARATION ET UTILISATION PHARMACEUTIQUE DE COMPOSITIONS À BASE D'EXTRAIT D'EUTERPE OLERACEA (ACAI)

(30) Priority: 18.07.2006 BR PI0604281
(43) Date of publication of application: 29.04.2009
(73) Proprietor: Universidade Do Estado Do Rio De Janeiro, CEP-20550-013 Rio de Janeiro RJ (BR)
(72) Inventor: SOARES DE MOURA, Roberto, Rio de Janeiro - RJ - CEP 22.410-090 (BR)
(74) Representative: CH Kilger Anwaltspartnerschaft mbB
(86) International application number: PCT/BR2007/000178
(87) International publication number: WO 2008/009084

(56) References cited:
- WO-A1-02/072118
- WO-A2-2004/084833
- BR-A- 0 107 227
- US-A1- 2004 002 132
- ROCHA ET AL: "Endothelium-dependent vasodilator effect of Euterpe oleracea Mart. (Acai) extracts in mesenteric vascular bed of the rat", VASCULAR PHARMACOLOGY, ELSEVIER, AMSTERDAM, NL, vol. 46, no. 2, 20 December 2006 (2006-12-20), pages 97-104, XP005810112, ISSN: 1537-1891, DOI: 10.1016/J.VPH.2006.08.411
- MATHEUS M E ET AL: "Inhibitory effects of Euterpe oleracea Mart. on nitric oxide production and iNOS expression", JOURNAL OF ETHNOPHARMACOLOGY, ELSEVIER SCIENTIFIC PUBLISHERS LTD, IE, vol. 107, no. 2, 22 March 2006 (2006-03-22), pages 291-296, XP025085812, ISSN: 0378-8741, DOI: 10.1016/J.JEP.2006.03.010 [retrieved on 2006-09-19]

## Description

### I) Field of Invention

The present invention deal with products that have anti-hypertensive properties, process to obtain products from plants belonging to *Palmae* family, more specifically to *Euterpe oleracea* specie, application of those products, process to obtain those products, more specifically a process to obtain a decoction from fruit of this plant, more specifically a process to obtain a decoction from fruits and/or stone of the fruit, more specifically a process to obtain a hydro-alcoholic extracts from those decoctions, more specifically, a process to obtain a lyophilized of those extracts, more specifically obtainment of pharmaceutical preparations and therapeutic indications in the treatment of vasoespastics and ischemic diseases and particularly arterial hypertension.

### II) Invention Antecedents

Ischemic diseases are a large problem in medicine particularly in cardiology where we can found vasospasm causing various circulatory problems as ischemic cardiopathy and vascular peripheral vascular diseases. Therefore search for new medicines that can reduce the contraction of vascular smooth muscle is a permanent goal in pharmacology.

Arterial hypertension is a disease with high prevalence among adult population and induces many deleterious effects in hypertensive patients, including cardiac, kidney and cerebral dysfunctions. Arterial hypertension is at the moment one of the largest causes of death. Therefore, pharmacological treatment that reduces the high level of arterial blood pressure and the cardiovascular complications from arterial hypertension is helpful for the patient and supported by health public agency. Usually the pharmacological treatment of hypertension is obtained with use of diuretics, beta blocking agents, inhibitors of the renin-angiotensin system, inhibitors of the sympathetic system and vasodilators compounds.

### III) Summary of the Invention.

The present invention is defined by claims 1-18. The present invention refers to a process to obtain products that have vasodilator and anti-hypertensive properties. Refer also of those products that are obtained from plants belonging to *Palmae* family, more specifically *Euterpe oleracea* specie, in particularly, to a process to obtain products obtained from decoctions obtained from the fruits and/or stones of the fruits. In particular a process for obtainment of the before mentioned products that means submit the fruits or the stones of the fruits of *Euterpe oleracea,* to an extraction to obtain a lyophilized and/or spray dried of those products.

More particularly, the present invention, refer the to a process to obtain products that have vasodilator and anti-hypertensive activity, that include utilization of the hole fruit or only the stone of the fruit, process to obtain a decoction from the whole fruit or only the stone of the fruit, extraction of the decoction with solvents, particularly solvents physiologically acceptable as ethanol, and process to obtain a lyophilized and/or spray dried of the pharmacological active products.

More particularly, refer also the present invention, methods to obtain products with vasodilator and anti-hypertensive properties as a lyophilized or spray dried, that means to submit the fruit or the stone of the fruit to an extraction with o physiological solvent, for instance water, as a decoction for 3 to 30 minutes. Also the present invention refers to o process of extraction of the decoction with ethanol for 5 to 15 days and posterior filtration and evaporation of the ethanol and also obtention of a lyophilized and/or spray dried with pharmacological activities. Furthermore, the present invention, as already mention above, refer to the before mentioned products per se and also the utilization of the before mentioned products and medicines containing the before mentioned products with vasodilator and anti-hypertensive activity.

### IV) Condensed description of the methodology used.

The present invention is supported by scientific data obtained in chemical and pharmacological experimentations. Below we give a brief view of the investigative process that supports the invention.

### IV.A) Method to obtain the liophyled and/or the spray dried of the hydroalcoholic extract of the fruit or stone of the fruits of Euterpe oleracea.

*Euterpe oleracea* fruits or only the stone of the fruits were washed in tap water and boiled in distilled water for 5 to 10 minutes and then minced and/or grinded, and then boiled and then extracted with ethanol (v: v) and macerated for 7 to 10 days and then the extract was filtered and the liquid phase is concentrated in a low-pressure rotator evaporator at approximately 40°C and then lyophilize or spay dried and kept in low temperature until the day of use..

### IV.B) Pharmacodynamic and pharmacotechnical methods.

The pharmacological activities of the products obtained from *Euterpe oleracea* was assessed by pharmacological tests that assessed the vasodilator effect in rats. The pharmacotechnical method refers the way to obtain capsules or tablets containing the lyophilized and/or the spray dried of the extracts obtained from *Euterpe oleracea* fruits or stone of the fruits.

### V.) Detailed description of the Invention.

In the ambit of the present invention, the fruits of *Euterpe oleracea* specie, before been submitted to the process of extraction, according to the invention, if not utilized after the harvest, can be stored for long periods at the temperature from +4 to -20° C.

### V.A) Method to obtain the dried residue, lyophilized or spray dried of the hydro alcoholic extract of the decoction of the whole fruits or decoction of the stone of the fruits of Euterpe oleracea.

The stones of the fruit of *Euterpe oleracea* are obtained by elimination of the skin of the whole fruit. The fruits or the stones are washed and boiled in distilled water for 3 to 9 minutes to obtain the decoction of the whole fruits or from the stones. After boiling the decoction are minced and/or grinded, and then boiled for another 5 minutes e extracted with ethanol 95% in a proportion that varies from 1:0.5 to 2:10, for instance 1:1. This mixture is again minced and/or grinded, and then macerated for a certain period of time (3 hours to 15 days) for instance 6 days at 4° C or at room temperature for instance 25° C under intermittent agitation. At the end of the maceration time the extract is filtered in a sieve with 0.1 to 1.0-mm pore, for instance 0.2 mm, being also filtered through gauze and finally filtered through a paper filter, for instance Whartman n.1. The semi-solid phase can be re-extracted on the same conditions already described for 1 to 3 times, for instance 2 times. The first liquid phase is kept inside a refrigerator at 4° C. The subsequent liquid phases are added to the first one. The liquid phase is concentrated in a low-pressure evaporator at a temperature of 35 to 65 ° C for instance 50° C and then lyophilized or spray dried.

### V.B) Pharmacological and Pharmacotechnique Methods.

The pharmacological activity of the products obtained from the fruits and/or the stones of *Euterpe oleracea* were assessed by pharmacological test in rats. The vasodilator effect was assessed in the isolated mesenteric vascular bed of the rat. The vasodilator effect of the extracts was assessed in the vascular mesenteric bed of the rat. Briefly, the rats were killed and the mesenteric artery was cannulated and perfused with Krebs solution with a pulsatile pump 4 mL per minute. The perfusion pressure was recorded. The vasodilator effect of the extract as assessed as reduction of the perfusion pressure induced by intra-arterial injections of the extract, during infusion of norepinephrine. The pharmacotechnique method refers to the method to obtain capsules or tablets containing the lyophilized or spray dried residue of the products obtained from *Euterpe oleracea.*

### VI.) General Observation.

The specific procedure described in item V.A to V.B above, were described not with the scope to limit, but wit the scope to illustrate the possibilities of realization of the present invention, with ambit is limited only by the claim annex.

### VII) Illustrative examples of the Invention.

Below are examples with the objective to illustrate and not to limit the present invention, which purpose, as mention above, has its delimitation's in the claim annex.

### VII.1) Method to obtain the dried residue, lyophilized and/or spray dried of the fruits of Euterpe oleracea decoction.

Approximately 200 g of *Euterpe oleracea* fruits were washed in tap water and boiled in 400 mL of distilled water during 5 minutes. After boiling the fruits were strongly minced and/or grinded and then 400 mL of ethanol was added to the decoction and again minced. This extract was macerate for 6 days and being also shaken for at least 1 hour per day. After maceration the decoction was filtered through a sieve with 0.2 mm pores and then filtered in gauze and then through a filter paper Whatman n.1. The liquid phase, obtained after filtration was concentrated under low pressure evaporator at 45° C, lyophilized and/or spray dried and kept at -20° C, until the day of use.

### VII.2) Method to obtain the dried residue, lyophilized and/or spray dried of the stone of Euterpe oleracea decoction.

Approximately 200 g of *Euterpe oleracea* fruits were washed in tap water and the stone was separated from the skin of the fruits. The stone was boiled in 400 mL of distilled water during 5 minutes. After boiling the stones were strongly minced and/or grinded and then 400 mL of ethanol was added to the decoction and again minced. This extract was macerate for 6 days and being also shaken for at least 1 hour per day. After maceration the decoction was filtered through a sieve with 0.2 mm pores and then filtered in gauze and then through a filter paper Whatman n.1. The liquid phase, obtained after filtration was concentrated under low pressure evaporator at 45° C, lyophilized and/or spray dried and kept at -20° C, until the day of use.

### VII.3). Example of biological tests performed with the products of the invention.

The vasodilator effect of the products were assesses in the isolated vascular mesenteric bed of the rat precontracted with norepinephrine.

### VII.3.1) Isolated Mesenteric Vascular Bed

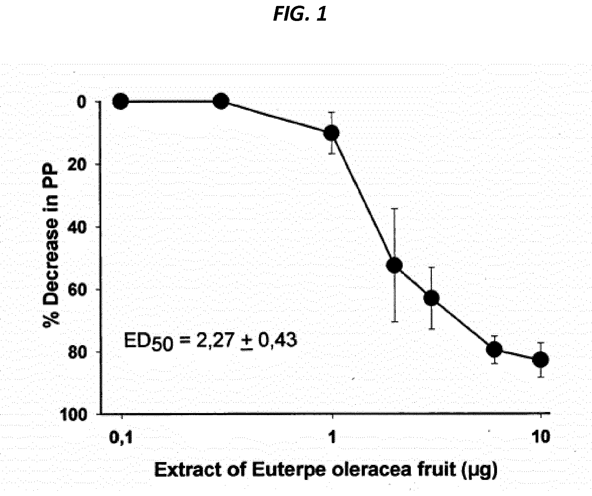

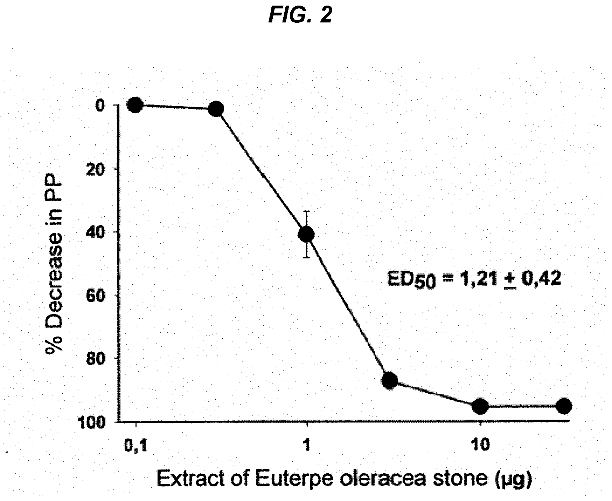

The rat superior mesenteric vascular bed (MVB) was isolated according to McGregor (1965). Briefly, male Wistar rats were killed with inhaled CO2 and the superior MVB was cannulated and perfused at a flow rate of 4ml min⁻¹ with a physiological salt solution (PSS) by a pulsatile pump (Lifecare Model 4, Abbott' Shaw). The PSS had the following composition (mM): NaCl 118, KCI 4.7, CaCl2 2.5, MgSO₄ 1.2, KH₂PO₄ 1.2, NaHCO₃ 25, EDTA 0.02, and glucose 11. The PSS (37°C) was bubbled with 95%O₂/5% CO₂. Perfusion pressure (PP) was measured with a transducer connected to a preamplifier and chart recorded. The lyophilizeds were either dissolved in PSS and administered as bolus injections directly into the perfusion stream (volume < 300 µl). The preparations were left to equilibrate for 30 minutes, and then injections of 120 µmol KCI were administered every 10 minutes until consistent responses were obtained. Lyophilized of the hydroacoholic extract (1 - 30 µg) was injected in bolus after the PP had been elevated (80-110 mmHg) with norepinephrine (NE; 6-30 µM) added to the perfusion fluid. When the pressor effect of NE reached a plateau, acetylcholine (ACh; 10 pmol) and nitroglycerin (NG; 1 nmol) were injected to test the endothelium-dependent and - independent responses before dose-response curves to lyophilized were obtained. The vasodilator effect of the extracts was expressed as a percent decrease in relation to the pressor effect of NE. The vasodilator effect of the lyophilized obtained from the fruit and from the stone is showed in Figures 1 and 2, in which Figure 1 comprises graphic of vasodilatator effect of hydro-alcoholic extract obtained from fruits of *Euterpe oleracea* in the isolated perfused vascular mesenteric bed of the rat preconcentrated norepinephrine. Ordinate: vasodilatation (%), expressed as a percentage decrease of the vasoconstriction induced by norepinephrine. Abscissa: doses of Extract of Euterpe oleracea fruits. Each point is presented as mean ± SEM. n=6 rats, and Figure 2 comprises graphic of vasodilatator effect of hydro-alcoholic extract obtained from stones of fruits of Euterpe oleracea in the isolated perfused vascular mesenteric bed of the rat, preconcentrated with norepinephrine. Ordinate: vasodilatation (%), expressed as a percentage decrease of the vasoconstriction induced by norepinephrine. Abscissa: doses of extract of Euterpe oleracea stones. Each point is presented as mean ± SEM. n=6 rats.

### VIII) Pharmacotechnical aspects of the preparation of capsules and tablets containing the Ivophilized obtained from the hydroalcoholic extract obtained from decoctions of fruit and stone of Euterpe oleracea.

The capsules and/or tablets containing 100 to 1000 mg of lyophilized of the hydroalcoholic extract obtained from decoctions of fruit and stone of Euterpe oleracea were obtained according to the usual Pharmacotechnical procedures. The capsules were obtained in order to contain 10 to 1000 mg, for instance 500 mg of the lyophilized plus cornstarch and colloidal silicon dioxide. Each capsule could have the following composition:

| | | |
|---|---|---|
| Lyophilized | 500 mg | 49.9% |
| Corn starch | 250 mg | 49.9% |
| Coloidal silicon dioxide | 0.5 mg | 0.2% |
| Total | 750.5 mg | 100% |

Cornstarch was added to complete the total mass of the capsule to approximately 750 mg. Colloidal silicon dioxide was used to adsorb humidity and to facilitate the preparation of the capsules.

### IX) Discussion of the invention in view of the closest state of the art documents

In MATHEUS M. et al.: "Inhibitory effects of Euterpe oleracea Mart. on nitric oxide production and iNOS expression". Journal of Pharmacology - Elsevier Scientific Publishers LTD, IE, vol. 107, no 2. 22/March/2006, the extract was obtained by maceration of *E. oleracea* fruits, flowers and spikes with ethanol at room temperature. Then, mincing and/or griding the fruits or only the stone of *Euterpe oleracea* boiling again during 5 minutes and extracting with ethanol and water (v:v) and macerating for 6 hours to 10 days.

In Matheus et al. it was demonstrated that an inhibitory effect on NO formation due to an inhibition of iNO synthase activity. WO2004/084833 - Murdock et al. - JUCARA AND AÇAI BASED DIETARY SUPLEMENTS. 07/10/2004. relates to compositions of Açai fruit and Jucara fruit with high antioxidant capability and cyclooxygenase-inhibitory activity, and their uses. It further provides for methods of making stable, palatable, freeze-dried, fruit-based dietary supplements from Açai fruit and Jucara fruit.

In Murdock et al. the method used is very similar to the North Brazil people (see WO2004084833 description on pag 47 paragraph [0322]). In such document, fruits of açaí are hulled for obtain its integral pulp. Therefore, the method described in WO2004084833 is a simple mechanical procedure performed at room temperature. The açaí integral pulp obtained therefore is not filtered, contains no only the liquid compounds soluble in water, but also solids or semi-solids components from açaí pulp that rapidly degraded in a few hours, unless it is frozen and freezing dried and no extraction in water and/or ethanol is performed.

Considering the therapeutic açaí juice obtained by the above discussed patent document teachings, the pharmaceutical activities described in the WO2004084833 is antioxidant and inhibitory action on COX-1 and COX-2.

## Claims

1. Process for obtaining a hydro-alcoholic extract from fruits of Euterpe oleracea **characterized by** the following steps:
a) Wash the fruits in tap water;
b) Submit the fruits of step (a) to a process of extraction in boiling water for 3 to 10 minutes to obtain a decoction;
c) Grind the decoction of step (b) with a mince for 3 to 10 minutes;
d) Boil the decoction for 3 to 10 minutes and
e) Extract the decoction obtained in step (d) with the solvent ethanol in the proportion of 1:1, v:v and mince strongly for 5 minutes and then macerate for 6 hours to 10 days at room temperature or at 4°C, as to obtain a hydro-alcoholic extract of the decoction.

2. Process according to claim 1 **characterized in that** the extraction of step (b) takes 5 minutes, mincing the decoction in step (c) takes 5 minutes and the decoction is boiled (d) for 5 minutes.

3. Process according to claim 1, **characterized in that** the time of maceration is 6 days.

4. Process according to claim 3 **characterized in that** the macerated decoction is filtered through a sieve with 1.0 mm pores and subsequently through filter paper.

5. Process according to claim 4 **characterized in that** the liquid phase is concentrated in a rotary evaporator at low pressure and at 40 to 60°C.

6. Process according to claim 5 **characterized in that** the alcohol free liquid phase after the concentration of the liquid phase is submitted to a process to obtain a lyophilized and/or a spray dried product, leading to the obtainment of a lyophilized or a spray dried product from the hydro-alcoholic extract with pharmacological activity.

7. Pharmaceutical preparations consisting of 10 to 1000 mg of a lyophilized and/or spray dried product obtained by a process according to claim 6 and also other inactive pharmacological components.

8. Pharmaceutical preparations according to claim 7, for use in a method of prevention and treatment of arterial hypertension and diseases induced by arterial hypertension.

9. The pharmaceutical preparation according to claim 8 for use in a method according to claim 8, which is used for human and veterinary applications.

10. Process for obtaining a hydro-alcoholic extract from stones of the fruits of Euterpe oleracea **characterized by** the following steps:
a) Wash the fruits in tap water;
b) Remove the skins of the fruits of step (a) to obtain the stones of the fruits;
c) Submit the stones of step (b) to a process of extraction in boiling water for 3 to 10 minutes to obtain a decoction of the stones;
d) Grind the decoction of step (c) for 3 to 5 minutes;
e) Boil the decoction for 3 to 10 minutes and
f) Extract the decoction obtained in step (e) with the solvent ethanol in the proportion of 1:1, v:v and grind and mince strongly for 5 minutes and then macerate for 6 hours to 10 days at room temperature or at 4°C, as to obtain a hydro-alcoholic extract of the decoction.

11. Process according to claim 10 **characterized in that** the extraction of step (c) takes 5 minutes, mincing the decoction in step (d) takes 5 minutes and the decoction is boiled in step (e) for 5 minutes.

12. Process according to claim 10, **characterized in that** the time of maceration is 6 days.

13. Process according to claim 12 **characterized in that** the macerated decoction is filtered through a sieve with 1.0 mm pores and subsequently through filter paper.

14. Process according to claim 13 **characterized in that** the liquid phase obtained after filtration is concentrated in a rotary evaporator at low pressure and at 40 to 60°C.

15. Process according to claim 14 **characterized in that** the ethanol free liquid phase after the concentration is submitted to a process for obtaining a lyophilized and/or a spray dried product, leading to the obtainment of a lyophilized or a spray dried product from the hydroalcoholic extract with pharmacological activity.

16. Pharmaceutical preparations consisting of 10 to 1000 mg of a lyophilized and/or spray dried product obtained by a process according to claim 15 and also other inactive pharmacological components.

17. Pharmaceutical preparations according to claim 16, for use in a method of the prevention and treatment of arterial hypertension and diseases induced by arterial hypertension.

18. The pharmaceutical preparation according to claim 17 for use in a method according to claim 17, which is used for human and veterinary applications.

## Patentansprüche

1. Verfahren zur Gewinnung eines hydroalkoholischen Extraktes aus Früchten von Euterpe oleracea, **gekennzeichnet durch** die folgenden Schritte:
a) Waschen der Früchte mit Leitungswasser;
b) die Früchte von Schritt (a) für 3 bis 10 Minuten einem Extraktionsprozess in siedendem Wasser unterziehen, um eine Abkochung zu erhalten;
c) Mahlen der Abkochung von Schritt (b) mit einem Hack für 3 bis 10 Minuten;
d) Kochen des Suds für 3 bis 10 Minuten und
e) die in Schritt (d) erhaltene Abkochung mit dem Lösungsmittel Ethanol im Verhältnis 1: 1, v: v extrahieren und 5 Minuten stark zermahlen und anschließend 6 Stunden bis 10 Tage bei Raumtemperatur oder bei 4 ° C mazerieren, um einen hydroalkoholischen Extrakt der Abkochung zu erhalten.

2. Verfahren gemäß Anspruch 1, **dadurch gekennzeichnet, dass** die Extraktion von Schritt (b) 5 Minuten dauert, das Zermahlen der Abkochung in Schritt (c) 5 Minuten dauert und die Abkochung für 5 Minuten gekocht wird (d).

3. Verfahren gemäß Anspruch 1, **dadurch gekennzeichnet, dass** die Mazerationszeit 6 Tage beträgt.

4. Verfahren gemäß Anspruch 3, **dadurch gekennzeichnet, dass** die mazerierte Abkochung durch ein Sieb mit 1,0 mm Poren und anschließend durch Filterpapier gefiltert wird.

5. Verfahren gemäß Anspruch 4, **dadurch gekennzeichnet, dass** die flüssige Phase in einem Rotationsverdampfer bei niedrigem Druck und bei 40 bis 60 °C konzentriert wird.

6. Verfahren gemäß Anspruch 5, **dadurch gekennzeichnet, dass** die alkoholfreie flüssige Phase nach dem das Konzentrat der flüssigen Phase einem Verfahren unterworfen wird, um ein lyophilisiertes und/oder ein sprühgetrocknetes Produkt zu erhalten, das zum Erhalt eines lyophilisierten oder sprühgetrockneten Produktes des hydroalkoholischen Extrakts mit pharmakologischer Aktivität führt.

7. Pharmazeutische Präparate, bestehend aus 10 bis 1000 mg eines lyophilisierten und/oder sprühgetrockneten Produkts, erhalten durch ein Verfahren gemäß Anspruch 6, und auch andere inaktive pharmakologische Komponenten.

8. Pharmazeutische Präparate gemäß Anspruch 7, zur Verwendung in einem Verfahren zur Vorbeugung und Behandlung von arteriellem Bluthochdruck und Krankheiten, die durch arterielle Hypertonie induziert werden.

9. Pharmazeutisches Präparat gemäß Anspruch 8 zur Verwendung in einem Verfahren gemäß Anspruch 8, das für humane und veterinärmedizinische Anwendungen verwendet wird.

10. Verfahren zur Gewinnung eines hydro-alkoholischen Extraktes aus Steinen der Früchte von Euterpe oleracea, **gekennzeichnet durch** die folgenden Schritte:
a) Waschen der Früchte mit Leitungswasser;
b) Entfernen der Häute der Früchte von Schritt (a), um die Steine der Früchte zu erhalten;
c) die Steine von Schritt (b) für 3 bis 10 Minuten einem Extraktionsprozess in siedendem Wasser unterziehen, um eine Abkochung der Steine zu erhalten;
d) Mahlen der Abkochung von Schritt (c) für 3 bis 10 Minuten;
e) Kochen des Suds für 3 bis 10 Minuten und
f) die in Schritt (e) erhaltene Abkochung mit dem Lösungsmittel Ethanol im Verhältnis 1: 1, v: v extrahieren und 5 Minuten stark zermahlen und anschließend 6 Stunden bis 10 Tage bei Raumtemperatur oder bei 4 °C mazerieren, um einen hydroalkoholischen Extrakt der Abkochung zu erhalten.

11. Verfahren gemäß Anspruch 10, **dadurch gekennzeichnet, dass** die Extraktion von Schritt (c) 5 Minuten dauert, das Zermahlen der Abkochung in Schritt (d) 5 Minuten dauert und die Abkochung für 5 Minuten gekocht wird (e).

12. Verfahren gemäß Anspruch 10, **dadurch gekennzeichnet, dass** die Mazerationszeit 6 Tage beträgt.

13. Verfahren gemäß Anspruch 12, **dadurch gekennzeichnet, dass** die mazerierte Abkochung durch ein Sieb mit 1,0 mm Poren und anschließend durch Filterpapier gefiltert wird.

14. Verfahren gemäß Anspruch 4, **dadurch gekennzeichnet, dass** die flüssige Phase, die nach der Filtration erhalten wurde, in einem Rotationsverdampfer bei niedrigem Druck und bei 40 bis 60 °C konzentriert wird.

15. Verfahren gemäß Anspruch 14, **dadurch gekennzeichnet, dass** die alkoholfreie flüssige Phase nach dem das Konzentrat der flüssigen Phase einem Verfahren unterworfen wird, um ein lyophilisiertes und/oder ein sprühgetrocknetes Produkt zu erhalten, das zum Erhalt eines lyophilisierten oder sprühgetrockneten Produktes des hydroalkoholischen Extrakts mit pharmakologischer Aktivität führt.

16. Pharmazeutische Präparate, bestehend aus 10 bis 1000 mg eines lyophilisierten und/oder sprühgetrockneten Produkts, erhalten durch ein Verfahren gemäß Anspruch 15, und auch andere inaktive pharmakologische Komponenten.

17. Pharmazeutische Präparate gemäß Anspruch 16, zur Verwendung in einem Verfahren zur Vorbeugung und Behandlung von arteriellem Bluthochdruck und Krankheiten, die durch arterielle Hypertonie induziert werden.

18. Pharmazeutisches Präparat gemäß Anspruch 17 zur Verwendung in einem Verfahren gemäß Anspruch 17, das für humane und veterinärmedizinische Anwendungen verwendet wird.

## Revendications

1. Procédé pour obtenir un extrait hydroalcoolique à partir de fruits d'Euterpe oleracea **caractérisé par** les étapes suivantes:
a) Laver les fruits dans l'eau du robinet;
b) Soumettre les fruits de l'étape (a) à un processus d'extraction dans l'eau bouillante pendant 3 à 10 minutes pour obtenir une décoction;
c) Broyer la décoction de l'étape (b) avec un hachis pendant 3 à 10 minutes;
d) Boil la décoction pendant 3 à 10 minutes et
e) Extraire la décoction obtenue à l'étape (d) avec le solvant éthanol dans la proportion de 1:1, v:v et hacher fortement pendant 5 minutes puis macérer pendant 6 heures à 10 jours à température ambiante ou à 4°C, comme pour obtenir un extrait hydroalcoolique de la décoction.

2. Procédé selon la revendication 1, **caractérisé en ce que** l'extraction de l'étape (b) prend 5 minutes, le fait de hacher la décoction dans l'étape (c) prend 5 minutes et la décoction est portée à ébullition (d) pendant 5 minutes.

3. Procédé selon la revendication 1, **caractérisé en ce que** le temps de macération est de 6 jours.

4. Procédé selon la revendication 3, **caractérisé en ce que** la décoction macérée est filtrée à travers un tamis de 1,0 mm et ensuite à travers du papier filtre.

5. Procédé selon la revendication 4, **caractérisé en ce que** la phase liquide est concentrée dans un évaporateur rotatif à basse pression et à 40 à 60°C.

6. Procédé selon la revendication 5, **caractérisé en ce que** la phase liquide sans alcool après la concentration de la phase liquide est soumise à un procédé pour obtenir un produit lyophilisé et/ou séché par pulvérisation, conduisant à l'obtention d'un produit lyophilisé ou séché par pulvérisation de l'extrait hydroalcoolique avec activité pharmacologique.

7. Préparations pharmaceutiques consistant en 10 à 1000 mg d'un produit lyophilisé et/ou séché par pulvérisation obtenu par un procédé selon la revendication 6 et aussi autres composants pharmacologiques inactifs.

8. Préparations pharmaceutiques selon la revendication 7, pour l'utilisation dans une méthode de prévention et de traitement de l'hypertension artérielle et des maladies induites par l'hypertension artérielle.

9. La préparation pharmaceutique selon la revendication 8, pour l'utilisation dans un procédé selon la revendication 8, qui est utilisée pour des applications humaines et vétérinaires.

10. Procédé pour obtenir un extrait hydroalcoolique à partir de pierres des fruits d'Euterpe oleracea **caractérisé par** les étapes suivantes:
a) Laver les fruits dans l'eau du robinet;
b) Enlevez les peaux des fruits de l'étape (a) pour obtenir les pierres des fruits;
c) Soumettre les pierres de l'étape (b) à un processus d'extraction dans l'eau bouillante pendant 3 à 10 minutes pour obtenir une décoction des pierres;
d) Broyer la décoction de l'étape (c) pendant 3 à 5 minutes;
e) Faire bouillir la décoction pendant 3 à 10 minutes et
f) Extraire la décoction obtenue à l'étape (e) avec le solvant éthanol à raison de 1:1, v:v et broyer et hacher fortement pendant 5 minutes puis macérer pendant 6 heures à 10 jours à température ambiante ou à 4 ° C, quant à obtenir un extrait hydroalcoolique de la décoction.

11. Procédé selon la revendication 10, **caractérisé en ce que** l'extraction de l'étape (c) prend 5 minutes, le fait de hacher la décoction dans l'étape (d) prend 5 minutes et la décoction est portée à ébullition dans l'étape (e) pendant 5 minutes.

12. Procédé selon la revendication 10, **caractérisé en ce que** le temps de macération est de 6 jours.

13. Procédé selon la revendication 12, **caractérisé en ce que** la décoction macérée est filtrée à travers un tamis de 1,0 mm et ensuite à travers du papier filtre.

14. Procédé selon la revendication 13, **caractérisé en ce que** la phase liquide obtenue après filtration est concentrée dans un évaporateur rotatif à basse pression et à 40 à 60°C.

15. Procédé selon la revendication 14, **caractérisé en ce que** la phase liquide exempte d'éthanol après la concentration est soumise à un procédé d'obtention d'un produit lyophilisé et/ou séché par pulvérisation, conduisant à l'obtention d'un produit lyophilisé ou séché par pulvérisation à partir de l'extrait hydroalcoolique avec activité pharmacologique.

16. Préparations pharmaceutiques consistant en 10 à 1000 mg d'un produit lyophilisé et / ou séché par pulvérisation obtenu par un procédé selon la revendication 15 et également d'autres composants pharmacologiques inactifs.

17. Préparations pharmaceutiques selon la revendication 16, pour l'utilisation dans une méthode de prévention et de traitement de l'hypertension artérielle et des maladies induites par l'hypertension artérielle.

18. Préparation pharmaceutique selon la revendication 17, pour l'utilisation dans un procédé selon la revendication 17, qui est utilisée pour des applications humaines et vétérinaires.
